# EUROPÄISCHE PATENTSCHRIFT

(11) **EP 1 954 352 B1**
(45) Veröffentlichungstag und Bekanntmachung des Hinweises auf die Patenterteilung: **15.01.2014**
(21) Anmeldenummer: 06806870.9
(22) Anmeldetag: 28.09.2006
(51) Int. Cl.: A61N 5/10, A61B 8/12

(54) **EINRICHTUNG FÜR DIE RÖNTGEN-BRACHYTHERAPIE**
DEVICE FOR X-RAY BRACHYTHERAPY
DISPOSITIF DE BRACHYTHÉRAPIE AUX RAYONS X

(30) Priorität: 24.11.2005 DE 102005056067
(43) Veröffentlichungstag der Anmeldung: 13.08.2008
(73) Patentinhaber: Siemens Aktiengesellschaft, 80333 München (DE)
(72) Erfinder: FEHRE, Jens, 91353 Hausen (DE); GRANZ, Bernd, 90522 Oberasbach (DE); LANSKI, Markus, 73433 Wasseralfingen (DE); NANKE, Ralf, 91077 Neunkirchen (DE)
(86) Internationale Anmeldenummer: PCT/EP2006/066845
(87) Internationale Veröffentlichungsnummer: WO 2007/060050

(56) Entgegenhaltungen:
- WO-A-99/39628
- WO-A1-2004/052460
- WO-A2-98/48899
- WO-A2-2006/061722
- DE-B3-102004 008 373
- US-B1- 6 416 492
- US-B1- 6 494 835
- US-B1- 6 721 392

## Beschreibung

Die Erfindung bezieht sich auf eine Einrichtung für die Röntgen-Brachytherapie.

Bei der Röntgen-Brachytherapie handelt es sich um eine therapeutische Behandlung mit Röntgenstrahlen, bei der die Röntgenquelle sehr nahe an das zu behandelnde Gewebe, beispielsweise ein Tumor oder eine Gefäßwand nach der Durchführung einer endovaskulären Dilatation, gebracht wird. Um die Röntgenquelle entweder ohne oder mit möglichst geringem invasiven Eingriff im Inneren eines Körpers mit Hilfe eines Katheters oder einer Sonde einführen zu können, wird eine miniaturisierte Röntgenquelle benötigt, wie sie beispielsweise aus der US 6,721,392 B1 bekannt ist. Diese ist am distalen Ende einer Sonde angeordnet, die intraoperativ beispielsweise in einem Tumor oder nach dessen Entfernung in einem Tumorbett positioniert wird, wie es beispielsweise in der PR-Information der Carl-Zeiss AG, Medizintechnik Innovation von Carl Zeiss AG, "Intraoperative Strahlentherapie mit dem INTRABEAM System von der Carl Zeiss AG, Stand September 2004, näher erläutert ist.

Aus der US 2003/0149327 A1 ist eine miniaturisierte Röntgenquelle bekannt, die in einem Katheter angeordnet ist, mit dem sie in die Körperhohlräume (Lumen) eingeführt werden kann, um von dort aus ausgewählte Gewebezonen aus unmittelbarer Nähe zu bestrahlen. Sie enthält eine um die Achse des Katheters drehbare Abschirmung, um die Röntgenstrahlen gezielt im Wesentlichen senkrecht zur Achse in einen ausgewählten Raumwinkel abzustrahlen. Mit einer im Katheter angeordneten optischen Beobachtungseinrichtung kann die Umgebung des Katheters betrachtet werden. Hierzu wird eine Lichtquelle verwendet, die nur den Teil der Oberfläche des Hohlraumes beleuchtet, der auch bestrahlt wird.

Auch bei der endovaskulären Brachytherapie mit einem in der Spitze eines Katheters angeordneten Beta- oder Gammastrahler ist es beispielsweise aus der DE 10 2004 008 373 B3 bekannt, im Katheter eine optische Beobachtungseinrichtung anzuordnen. Hierzu wird ein Brachytherapie-Katheter mit einem auf der Grundlage der optischen Kohärenztomographie (OCT) arbeitenden OCT-Katheter zu einer Einheit integriert.

Wesentlich für den therapeutischen Erfolg ist, dass die von der Röntgenquelle in ein Bestrahlungsgebiet außerhalb des Katheters abgestrahlten Röntgenstrahlen weitgehend ausschließlich auf das zu behandelnde Gewebe, beispielsweise den Tumor, auftreffen, um eine möglichst geringe Belastung des daneben befindlichen gesunden Gewebes sicherzustellen. Dies erfordert eine präzise Positionierung des Bestrahlungsgebietes, d.h. eine präzise Positionierung und Ausrichtung der Röntgenquelle bzw. des Raumwinkels, in den die Röntgenstrahlen austreten.

Positionierungsverfaren sind aus z.B. WO 99/39628 und WO 2004/052460 bekannt.

Der Erfindung liegt nun die Aufgabe zu Grunde, eine Einrichtung für die Röntgen-Brachytherapie mit einer in das Innere eines Körpers einführbaren Sonde anzugeben, mit der eine genaue Positionierung des Bestrahlungsgebietes möglich ist.

Hinsichtlich der Einrichtung wird die genannte Aufgabe gelöst mit einer Einrichtung mit den Merkmalen des Patentanspruches 1. Gemäß diesen Merkmalen enthält die Einrichtung eine in das Innere eines Körpers einführbare Sonde, die an ihrem distalen Ende eine Röntgenquelle aufweist, die Röntgenstrahlen in ein Bestrahlungsgebiet außerhalb der Sonde abstrahlt, wobei in der Sonde ein Ultraschall-Array zum Erzeugen eines zumindest einen Teil des Bestrahlungsgebietes wiedergebenden Ultraschallbildes angeordnet ist. Dies ermöglicht eine genaue Positionierung des Bestrahlungsgebietes, d.h. eine genaue Positionierung der Röntgenquelle und des Raumwinkelbereiches, in dem die von der Röntgenquelle erzeugten Röntgenstrahlen abgestrahlt werden.

Unter dem Begriff "Sonde" ist im Folgenden allgemein ein Instrument zu verstehen, das in das Innere eines Körpers eingebracht werden kann. Dies kann sowohl ein Katheter im engeren Sinn sein, das in Hohlräume des Körpers (transluminal) eingeführt wird, als auch ein innerhalb einer Gewebezone (perkutan oder intertsitiell) platzierbares nadelähnliches Instrument sein.

Wenn in der Sonde zur Einstellung des Bestrahlungsgebietes eine relativ zur Röntgenquelle bewegliche Abschirmung angeordnet ist, kann das Bestrahlungsgebiet auch bei ruhender Sonde flexibel eingestellt werden.

Die Einrichtung umfasst eine Wiedergabeeinrichtung zur Darstellung des Ultraschallbildes und zum Kenntlichmachen des Bestrahlungsgebietes im Ultraschallbild. Dies ermöglicht eines besonders einfache und anschauliche Positionierung des Bestrahlungsgebietes.

Wenn außerdem in das im Ultraschallbild wiedergegebene Bestrahlungsgebiet Linien gleicher Dosisleistung eingeblendet sind, kann gezielt die an verschiedenen Orten des Bestrahlungsgebietes erforderliche Dosisleistung eingestellt werden.

In einer bevorzugten Ausgestaltung wird im Ultraschallbild auch die Lage der Röntgenquelle angezeigt.

Von besonderem Vorteil ist es, wenn ergänzend zum Ultraschallbild mit Hilfe einer in oder an der Sonde angeordneten optischen Beobachtungseinrichtung ein optisches Bild einer zumindest einen Teil des Bestrahlungsgebietes enthaltenden Umgebung der Sonde erzeugt wird. Dadurch wird das korrekte Navigieren der Sonde zusätzlich insbesondere dann erleichtert, wenn im optischen Bild das Bestrahlungsgebiet kenntlich gemacht wird.

Eine Einrichtung gemäß der Erfindung ist insbesondere zum Einführen in eine Harnröhre oder einen Harnleiter und zur therapeutischen Behandlung eines Prostata-, Blasen- oder Nierentumors geeignet.

Zur weiteren Erläuterung der Erfindung wird auf das Ausführungsbeispiel der Zeichnung verwiesen. Es zeigen:
- Fig. 1: eine Einrichtung gemäß der Erfindung in einer schematischen Prinzipdarstellung,
- Fig. 2: ein Ultraschallbild, in das das Bestrahlungsgebiet eingeblendet ist,
- Fig. 3: ein optisches Bild aus der Umgebung der Sonde, in dem ebenfalls das Bestrahlungsgebiet kenntlich gemacht ist.

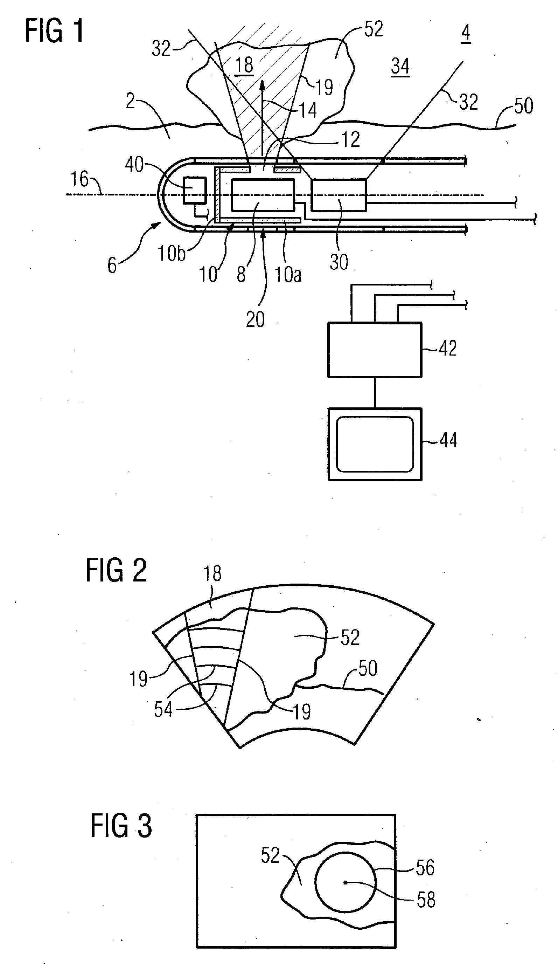

Gemäß Fig. 1 ist in einen Hohlraum (Lumen) 2 eines Körpers 4 bei dem es sich um eine Urethra (Harnröhre) oder einen Ureter (Harnleiter) handeln kann, eine Sonde 6, im Beispiel ein Katheter, eingeführt, in der an ihrem distalen Ende eine Röntgenquelle 8 angeordnet ist. Der Röntgenquelle 8 ist eine Abschirmung 10 zugeordnet, die im Ausführungsbeispiel einen zylindrischen Teil 10a enthält, der in seinem Umfang mit einer Blende oder Öffnung 12 versehen ist, durch die Röntgenstrahlen 14 senkrecht zur Längsachse 16 der Sonde 6, d.h. radial in ein durch die Form der Öffnung 12 und deren Abstand zur Anode der Röntgenquelle 8 definiertes, beispielsweise kegelförmiges Bestrahlungsgebiet 18, austreten können, das in der Figur durch eine Schraffur hervorgehoben und durch Begrenzungslinien 19 kenntlich gemacht ist.

Der zylindrische Teil 10a der Abschirmung 10 ist innerhalb der Sonde 6 um deren Längsachse 16 drehbar angeordnet, so dass das Bestrahlungsgebiet 18 ebenfalls um diese Längsachse 16 geschwenkt werden kann. Hierzu ist die Außenwand der Sonde 6 mit einem ringförmig umlaufenden, für Röntgenstrahlen 14 durchlässigen Fenster 20 versehen. Die Sonde 6 kann aber auch in einem größeren Bereich durchlässig für Röntgenstrahlen 14 sein und beispielsweise eine aus einem polymeren Werkstoff bestehende Außenwand aufweisen.

Die Abschirmung 10 weist an einer ihrer Stirnseiten eine Stirnplatte 10b auf, die mit einer in der Figur nicht näher dargestellten verschließbaren Blende versehen ist, mit der es möglich ist, wahlweise Röntgenstrahlen 14 in Richtung der Längsachse 16 abzustrahlen. In diesem Fall ist entweder ein beweglicher Verschluss vorgesehen, mit dem die Öffnung 12 verschlossen werden kann oder eine Zusatzabschirmung, die in der Sonde 6 derart angeordnet ist, dass die Öffnung 12 im Bereich dieser Abschirmung positioniert werden kann. Dies kann beispielsweise dadurch realisiert werden, dass sich das Fenster 20 nicht über den gesamten Umfang erstreckt.

In unmittelbarer Nähe der Röntgenquelle 8 ist ein Ultraschall-Array 30 angeordnet, das ein Ultraschallbild aus einem durch Begrenzungslinien 32 veranschaulichten Objektgebiet 34 erzeugt, das zumindest einen Teil des Bestrahlungsgebietes 18 überlagert.

Die Sonde 6 enthält außerdem eine schematisch angedeutete optische Beobachtungseinrichtung 40, mit der ein Objektbereich beobachtet werden kann, der zumindest einen Teil des Bestrahlungsgebietes 18 wiedergibt.

Die Sonde 6 ist an eine Steuer- und Auswerteeinrichtung 42 angeschlossen, mit der die Röntgenquelle 8, das Ultraschall-Array 30 und die Beobachtungseinrichtung 40 gesteuert und die vom Ultraschall-Array 30 und von der Beobachtungseinrichtung 40 übermittelten Signale ausgewertet werden, so dass sie als Ultraschallbild bzw. als optisches Bild in einer Wiedergabeeinrichtung 44, beispielsweise ein Monitor, dargestellt werden können.

Im Beispiel der Fig. 1 befindet sich im Bereich der Wand 50 des Hohlraumes 2 eine therapeutisch zu behandelnde Gewebezone 52, beispielsweise ein Tumor, insbesondere ein Prostatatumor, ein Blasentumor oder ein Nierentumor, die mit den Röntgenstrahlen 14 bestrahlt werden soll.

Im Ultraschallbild der Fig. 2 ist diese Gewebezone 52 sowie die Wand 50 schematisch wiedergegeben. In diesem Ultraschallbild ist nun die Schnittfläche des Bestrahlungsgebietes 18 mit der vom Ultraschall-Array erfassten Objektebene beispielsweise durch Wiedergabe seiner seitlichen Begrenzungslinien 19 kenntlich gemacht. Das auf diese Weise in das Ultraschallbild eingeblendete Bestrahlungsgebiet 18 ermöglicht eine korrekte Positionierung der Sonde bzw. der Röntgenquelle.

In das Ultraschallbild eingeblendet sind außerdem etwa kreisbogenförmige Linien 54 gleicher Dosisleistung, die dem Therapeuten die aktuelle lokale Dosisleistung anzeigen. Diese Linien 54 befinden sich beispielsweise untereinander in einem Abstand, der dem Abfallen der Dosisleistung auf jeweils 1/e entspricht. Dies sind bei einer Röntgenstrahlung mit einer mittleren Energie von 20 keV im Gewebe etwa 1,2 cm. Der Therapeut kann dann durch Änderung der Betriebsparameter der Röntgenquelle (Anodenstrom, Beschleunigungsspannung) die erforderliche Dosisleistung einstellen. Diese Linien 54 gleicher Dosisleistung verschieben sich dann im Ultraschallbild entsprechend der sich bei der Variation der Betriebsparameter ändernden Dosisleistung.

Gemäß Fig. 3 wird auch ein optisches Bild aus der Umgebung der Sonde erzeugt, in dem bei entsprechender Beleuchtung die Wand 50 des Hohlraumes sichtbar ist. Wird zusätzlich ein Leuchtstoff appliziert, beispielsweise unmittelbar mit Hilfe der Sonde, der sich bevorzugt in der Gewebezone 52 anlagern, wenn es sich bei dieser um einen Tumor handelt, und der beispielsweise durch die zur Beleuchtung verwendeten Lichtquelle angeregt wird und Fluoreszenzlicht im sichtbaren Bereich emittiert, kann der Tumor 52 zumindest in seinem Oberflächenbereich mit dem er an dem Hohlraum angrenzt auch im optischen Bild sichtbar gemacht werden. Wird zusätzlich die Einhüllende des auf die Oberfläche der Wand 50 auftreffenden Röntgenstrahlbündels als Kreislinie 56 und dessen Mittenachse als Punkt 58 eingeblendet, kann die exakte Positionierung auch mit Hilfe des optischen Bildes ergänzend zu der Positionierung mit dem Ultraschallbild vorgenommen werden, da in diesem Fall eine Bildinformation aus einer anderen, beispielsweise zur Bildebene des Ultraschallbildes senkrechten Ebene vorliegt.

Die Erfindung ist anhand eines in den Hohlraum eines Körpers eingeführten Katheters dargestellt. Grundsätzlich ist die Erfindung auch für Sonden geeignet, die unmittelbar in das Gewebe eingeführt werden, wie das bei der eingangs erläuterten invasiven Nachbehandlung eines Tumorbettes eines vorher entfernten Tumors der Fall ist. Bei der zu behandelnden Gewebezone kann es sich auch um eine Gefäßwand handeln, die nach der Durchführung einer Dilatation zur Verringerung der Restenoserate bestrahlt werden soll.

## Patentansprüche

1. Einrichtung für die Röntgen-Brachytherapie mit einer in das Innere eines Körpers (4) einführbaren Sonde (6), die an ihrem distalen Ende eine Röntgenquelle (8) aufweist, die Röntgenstrahlen (14) in ein Bestrahlungsgebiet (18) außerhalb der Sonde (6) abstrahlt, und mit einem in der Sonde (6) angeordneten Ultraschall-Array (30) zum Erzeugen eines zumindest einen Teil des Bestrahlungsgebietes (18) wiedergebenden Ultraschallbildes, sowie mit einer Wiedergabeeinrichtung (44) zur Darstellung des Ultraschallbildes und zum Kenntlichmachen des Bestrahlungsgebietes (18) im Ultraschallbild.

2. Einrichtung nach Anspruch 1, bei der in der Sonde (6) zur Einstellung des Bestrahlungsgebietes (18) eine relativ zur Röntgenquelle (8) bewegliche Abschirmung (10) angeordnet ist.

3. Einrichtung nach Anspruch 1 oder 2, bei der in das im Ultraschallbild wiedergegebene Bestrahlungsgebiet (18) Linien (54) gleicher Dosisleistung eingeblendet sind.

4. Einrichtung nach Anspruch 2 oder 3, bei der im Ultraschallbild die Lage der Röntgenquelle (8) angezeigt wird.

5. Einrichtung nach einem der vorhergehenden Ansprüche, bei der die Sonde (6) eine optische Beobachtungseinrichtung (40) zum Erzeugen eines optischen Bildes einer zumindest einen Teil des Bestrahlungsgebietes (18) umfassenden Umgebung der Sonde (6) enthält.

6. Einrichtung nach Anspruch 5, bei der im optischen Bild das Bestrahlungsgebiet (18) kenntlich gemacht ist.

## Claims

1. Device for x-ray brachytherapy comprising a probe (6) which can be inserted into the inside of a body (4), said probe having an x-ray source (8) at the distal end thereof that radiates x-rays (14) into an exposure area (18) outside of the probe (6) and an ultrasound array (30) located in the probe (6) for generating an ultrasound image representing at least a portion of the exposure area (18) and a display device (44) for displaying the ultrasound image and for allowing identification of the exposure area (18) in the ultrasound image.

2. Device according to claim 1, in which a shield (10) that is moveable relative to the x-ray source (8) is arranged in the probe (6) so as to adjust the exposure area (18).

3. Device according to claim 1 or 2, in which lines (54) of the same dose rating are shown in the exposure area (18) reproduced in the ultrasound image.

4. Device according to claim 2 or 3, in which the position of the x-ray source (8) is displayed in the ultrasound image.

5. Device according to one of the preceding claims, in which the probe (6) contains an optical observation device (40) for generating an optical image of an environment of the probe (6) encompassing at least one portion of the exposure area (18).

6. Device according to claim 5, in which the exposure area (18) is made known in the optical image.

## Revendications

1. Dispositif de brachythérapie aux rayons X comprenant une sonde ( 6 ) qui peut être introduite à l'intérieur d'un corps ( 4 ) et qui a, à son extrémité distale, une source ( 8 ) de rayons X qui émet des rayons ( 14 ) X dans une zone ( 18 ) d'irradiation à l'extérieur de la sonde ( 6 ) et comprenant une matrice ( 30 ) à ultrason disposée dans la sonde ( 6 ) pour la production d'une image d'ultrason reproduisant au moins une partie de la zone ( 18 ) d'irradiation, ainsi qu'un dispositif ( 44 ) de restitution pour la représentation de l'image d'ultrason et pour rendre reconnaissable la zone ( 18 ) d'irradiation dans l'image d'ultrason.

2. Dispositif suivant la revendication 1, dans lequel un écran ( 10 ) mobile par rapport à la source ( 8 ) de rayons X est monté dans la sonde ( 6 ) pour le réglage de la zone ( 18 ) d'irradiation.

3. Dispositif suivant la revendication 1 ou 2, dans lequel des lignes ( 54 ) de même débit de dose sont incrustées dans la zone ( 18 ) d'irradiation reproduite dans l'image d'ultrason.

4. Dispositif suivant la revendication 2 ou 3, dans lequel la position de la source ( 8 ) de rayon X est indiquée dans l'image d'ultrason.

5. Dispositif suivant l'une des revendications précédentes, dans lequel la sonde ( 6 ) comporte un dispositif ( 40 ) d'observation optique pour la production d'une image optique d'un environnement de la sonde ( 6 ) comprenant au moins une partie de la zone ( 18 ) d'irradiation.

6. Procédé suivant la revendication 5, dans lequel la zone ( 18 ) d'irradiation est rendue reconnaissable dans l'image optique.
